(19) Europäisches Patentamt
European Patent Office
Office européen des brevets

(11) Publication number: 0 099 169 B1

# (12) EUROPEAN PATENT SPECIFICATION

(45) Date of publication of patent specification: 20.07.88

(51) Int. Cl.$^4$: A 61 C 5/10, A 61 C 13/00, A 61 K 6/00, A 61 K 6/08, A 61 K 6/02

(21) Application number: 83302256.9

(22) Date of filing: 20.04.83

## (54) Manufacture and repair of dental appliances.

(30) Priority: 20.04.82 US 370215

(43) Date of publication of application:
25.01.84 Bulletin 84/04

(45) Publication of the grant of the patent:
20.07.88 Bulletin 88/29

(84) Designated Contracting States:
AT BE CH DE FR GB LI NL

(56) References cited:
EP-A-0 047 097
DE-B-2 162 608
DE-C- 738 163
DE-C- 744 633
GB-A-1 115 544
US-A-2 558 139
US-A-3 889 385
US-A-3 997 637
US-A-4 125 442
US-A-4 247 575
US-A-4 295 941
BRITISH DENTAL JOURNAL, vol. 149, 1980
J.W. McLEAN:"Aesthetics in restorative dentistry: the challenge for the future", pages 368-373

(73) Proprietor: DENTSPLY INTERNATIONAL, INC.
570 West College Avenue P.O. Box 872
York Pennsylvania 17405 (US)

(72) Inventor: Milnes, Ian M.
114 East Keller Street
Mechanicsburg, PA 17055 (US)
Inventor: Tateosian, Louis H.
209 Reynolds Mill Road
York, PA 17403 (US)

(74) Representative: Lamb, John Baxter et al
MARKS & CLERK 57/60 Lincoln's Inn Fields
London WC2A 3LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 099 169 B1

Courier Press, Leamington Spa, England.

**Description**

This invention is directed to methods for the manufacture and repair of dental appliances especially crowns and bridges. More particularly, a system is provided whereby dental appliances may be fabricated or repaired in the mouth or externally. Such methods are provided which provide dental appliances which are physically and aesthetically superior to those presently known. In addition, this invention is directed to materials useful for the preparation of dental appliances and for the repair thereof which have the ability to bond to metallic underlayments or substrates with improved tenacity.

The fracture of porcelain from fixed dental appliances such as crowns and bridge work has been a problem for dentists for many years. The repair of such prostheses has been accomplished in the past in two ways. Typically, a prosthesis is removed from the patient's mouth and repaired in a dental laboratory. This procedure may be quite painful and traumatic to the patient. Further, damage to existing dentition and/or to the dental appliance itself is frequently encountered. This procedure requires extensive amounts of pactitioner time in the mouth with attendant expense. In the alternative, it has been known to repair dental appliances while they remain fixed within a patient's mouth. While this effects a substantial savings in working time in the mouth, prior methods and materials have provided only mediocre aesthetic results having a relatively short lifetime.

It will be appreciated that the construction and repair of dental appliances necessarily involve the bonding of materials to dental metals. Such prosthetic devices generally comprise shaped metallic underlayments which are fabricated from a metallic species or alloy having good strength, durability, etc. and also compatability with the oral environment. Such alloys which frequently include high gold alloys, are generally inert and provide a difficult bonding surface. Accordingly, prior art materials and methods which involve the application of polymerizable adhesives and other materials to the surface of dental alloys have had poor bond strengths and concomitant poor durabilities. Efforts to overcome these shortcomings have not been successful. For example, a cold-setting ethoxylated bisphenol-A dimethacrylate resin sold by the Denmat Company incorporates certain silanous materials such as alpha-methacryloxypropyltrimethoxysilane and other materials as adhesion promoters for application to dental metals. Such material does not provide permanent bonding to dental alloys however. The product "Fusion"® is a two component material, (believed to be a silane-based product) which has been sold as an adhesion promoter. The product "Metalit"® is a cyanoacrylate based system which, again, is believed to provide only a temporary bond to dental alloys in the oral environment. It is believed that when applied to dental alloys, the foregoing materials bond only weakly to the metal. It is further believed that oral fluids are capable of hydrolyzing or otherwise destroying this bonding causing a loss of adhesion to the metal underlayment or substrate.

U.S. Patent No. 3,889,385 discloses dental opaqueing materials comprising copolymers of methyl methacrylate and acrylonitrile in a solvent medium. Small amounts of acrylic or methacrylic acid may be included in these compositions as may certain silanes. The composition of US—A—3889385 and used to form opaque coating on metal substrates.

U.S. Patent No. 3,997,637 discloses methods for making tooth reconstructions employing electrodeposition techniques. U.S. Patent No. 4,125,442 discloses the preparation of composite tooth reconstructions by bonding porcelain over a basis metal coated with electrolytically deposited metal to improve the bond strength of the porcelain to the metal.

U.S. Patent No. 4,247,575 is directed to methods for electroless plating of silver onto tooth structures, especially dentin.

U.S. Patent No. 4,295,951 provides improved porcelain coated metal dental articles through electroplating gold over a non-precious metal substrate, adding finely divided gold particles thereto and firing porcelainic materials thereover.

McLean in "Aesthetics and Restorative Dentistry: The Challenge for the Future" British Dent. Journal Vol. 149, pp. 368 *et seq* (1980) suggests the coating of nickel-chrome with noble metals such as gold, platinum or rhodium by electroplating followed by plating to improve the receptivity of the metal to porcelain coatings.

None of the foregoing discloses or suggests the novel methods and materials of the present invention.

This invention provides methods for the manufacture of dental appliances and prostheses and for their repair.

According to the invention there is provided a method for the construction of a dental appliance for use in an oral environment comprising:

(a) providing a shaped underlayment of a first metallic species generally inert with respect to the oral environment;

(b) plating onto at least a portion of the underlayment a layer of a second metallic species;

(c) applying to said surface portion an amount sufficient substantially to obscure the layer, of a polymerizable opaqueing agent; and

(d) polymerizing said opaqueing agent; characterized in that a surface portion of the layer of the second metallic species is oxidized, in a step (b'), prior to step (c) and that at least one of steps (b'), (c) and (d) is performed intraorally.

It is preferred that an adhesion promoter be employed in accordance with the present methods. Thus,

an adhesion promoter may be applied to the second metallic layer prior to the application of the opaqueing agent. In the alternative, the adhesion promoter may be included as a component of the opaqueing agent. The opaqued portion is well adapted for the application of a cosmetic or aesthetic veneer thereto.

It is preferred that the opaqueing agent be curable or polymerizable through exposure to actinic radiation such as to visible light. It is also preferred that the adhesion promoter, if used, comprise a material having dual chemical functionality. Thus, a first functionality comprises one or more polymerizable groups while the second functionality is selected to be capable of bonding with the surface of the second metallic species either ionically, coordinately, or covalently.

The foregoing methods and materials are suitable for construction of dental articles either within the mouth or externally. Thus, crowns, bridges, temporary bridges, and a wide variety of other oral prosthetics may be so constructed or repaired.

The present invention is directed to methods for the manufacture and repair of dental appliances and prostheses. In accordance with the present invention there is provided a shaped, metallic underlayment which will generally comprise a dental alloy material. A layer of a second metallic species is then plated onto at least a portion of the underlayment and oxidised to provide a surface more hospitable toward bonding with polymerizable species. To the plated layer is then applied an amount sufficient substantially to obscure the layer of a polymerizable opaqueing agent which is subsequently polymerized. It is preferred that the layer of a second metallic species be treated with an adhesion promoter prior to applying the opaqueing agent. In the alternative, it is possible to combine an adhesion promoter together with the opaqueing agent or to apply the two species concurrently.

The foregoing procedures provide a surface of a polymerized material on the surface of a shaped metallic underlayment. By virtue of the interposition of a second metallic species arrived at through a plating technique between the metallic underlayment and the polymerized material, superior bonding of the polymerized species to the underlayment takes place. The layer of polymerized material is designed to subsequently obscure the layer of second metallic species so that the aesthetic detraction of an intraoral metallic mass is avoided. The polymerized layer is well suited for application of additional species thereto. Accordingly, aesthetic veneers and the like may be applied to this layer to effect the construction of a wide variety of dental appliances such as crowns, bridges, and other devices. Alternatively, additional layers of polymerizable materials may be built up on the surface of the metallic underlayment and either smoothed or sculpted to result in a pleasing appearance for a dental object formed thereby.

The shaped, metallic underlayment which is provided during the course of the practice of the methods of this invention may comprise any of a wide variety of metallic species which are known for use in the preparation of dental appliances and prostheses. Accordingly, metallic forms for crowns, bridges oral structures, artificial teeth, and a wide variety of other intraoral metallic foundation objects or underlayments may be so employed. Those skilled in the art will appreciate that, in general, such underlayments will comprise one of a number of dental alloys or metal materials such as for example, gold, nickel, silver, platinum etc. together with alloys of the foregoing, and a wide variety of other metallic species. In general, such shaped metallic objects will be formulated so as to have little or no physiological effects in the mouth of a patient. Accordingly, such materials are generally considered to be inert and possessed of a relatively passive metal surface.

In accordance with the present invention, at least a portion of the shaped, metallic underlayment is plated with a layer of a second metallic species. The second metallic species is selected to be one which forms stronger bonds with the opaqueing agents of the present invention than do the metallic underlayments. Accordingly, it is desired to provide such second metallic species which are less inert to bonding than are the dental alloys comprising the shaped metallic underlayments. It is preferred that such second metallic species be materials such as tin, zinc, alloys thereof, and similar species which are known to form a highly oxidized surface. The second metallic species may also be applied sequentially through a plurality of plating steps onto the shaped metallic underlayment.

The plating may take place either through an electrolytic process or through an electroless method. Those skilled in the art will appreciate that each of the foregoing plating techniques may be employed intraorally using presently available commercial methods. It will be appreciated that the nature of metallic plating is such that the bond between the plated layer of a second metallic species and the metallic underlayment will be quite strong due to metallic bonding and possibly the formation of a complex metallic alloy layer at the interface of the two metals. The surface of the second metallic layer outward of the underlayment should be present in a relatively highly oxidized form to promote bonding with subsequently applied opaqueing materials. Accordingly treatment of the layer with peroxides or otherwise may, optionally, be employed to promote such oxidation. It is convenient to plate onto the metallic underlayment an amount of a second metallic species which is sufficient to substantially completely cover the portion of the underlayment thus plated. Substantial quantities of the second metallic species are not presently believed to be beneficial although they may be applied if desired.

The plated surface is contacted with an amount sufficient to substantially obscure the plate layer of a polymerizable opaqueing agent. Such opaqueing agents may be selected from a wide variety of polymerizable, resin-based materials as are known to those skilled in the art. See in this regard U.S. Patent 3,889,385 and unfilled or sparingly filled versions of compositions disclosed in DE—A—3133008 and European Patent Publications Nos. 0047097 and 0064834, all assigned to the assignee of this invention,

which disclose a wide variety of resin-based materials which may be so employed. It is preferred that the foregoing materials be filled and/or pigmented so as to improve the opacity thereof. For example, addition of titania, alumina, Schott glass and other opacification agents is preferred. The opaqueing agent is applied in amounts sufficient to substantially obscure the metallic layer to which it is applied. In general, amounts of opaqueing agent sufficient to produce a layer of polymerizable material of from about 0.5 to 2.0 mm is sufficient. More may be used, however, such as when sculpting or smoothing to a finished surface if desired.

The foregoing opaqueing agents may be polymerizable through the application of thermal energy, radiation, or actinic light. Of the foregoing, it is preferred that such materials be formulated to be polymerizable through the application of actinic light, especially visible light. Accordingly, sensitizing systems or catalysts suitable for promoting the thermal or photochemical polymerization of the opaqueing agent are preferably included therein. According to a still more preferred embodiment, a visible light sensitizing agent comprising camphoroquinone and an amine reducing agent are employed to render a polymerizable resin system polymerizable through exposure to visible radiation.

Following the application of the polymerizable opaqueing agent, the same is polymerized through the application of either heat, radiation or actinic light depending upon the chosen formulation for the opaqueing agent. Thus, it is preferred that the opaqueing agent be exposed to, for example, visible light and that the opaqueing agent be formulated so as to be sensitive thereto. The foregoing system is especially preferred for intra-oral employment of the present methods as the exposure of living tissue to visible light is considered to be innocuous.

In accordance with another preferred embodiment of the present invention, an adhesion promoting agent is also employed. Thus, the portion of the shaped metallic underlayment which has been plated with a layer of a second metallic species is caused to be more receptive to bonding with the opaqueing agent by application of such adhesion promotion agent. In the alternative, the adhesion promotion agent may be included within the opaqueing agent formulation itself; the two may be applied concurrently.

The adhesion promotion agent generally comprises a di- or polyfunctional material having two types of functionalities. Such materials preferably comprise chemical species having one or more polymerizable functionalities, especially ethylenic unsaturations, together with one or more functionalities capable of either ionically, covalently or coordinately bonding with the surface of the second metallic species. Accordingly, the adhesion promotion agents may be represented by the following general formula:

I $$(X)_m—R—(Y)_n$$

wherein R is a hydrocarbyl group having from 1 to 20 carbon atoms, X is a functionality adapted to bond to the surface of the second metallic species, Y is a polymerizable group and n and m are integers having a value from about 1 to about 3. It is preferred that the polymerizable functionalities, Y, be ethylenic unsaturations which are capable of interpolymerizing with the polymerizable materials of the opaqueing agent. Accordingly, it is preferred that such materials be acrylates, methacrylates and related species. Alternatively, however, such functionalities may be any of the polymerizable functionalities which are disclosed for use in connection with any of the foregoing patent specifications. The metallic surface bonding functionalities, X, may be any of a number of functionalities which are capable of relatively strong bonding or association with the surface of the second metallic species.

The bonding functionalities may be selected from ionic species such as phosphates, sulphates and the like which may form inorganic ester bonds with oxide or hydroxylic linkages on the surfaces of the second metallic species. Preferred among this class of promoters are glycerophosphoric acid dimethacrylate, diacrylate, and similar species; pentaerythritol trimethacrylate phosphate, related acrylates; and similar sugar-type acrylics.

In an alternative embodiment, the bonding functionalities may comprise any of a number of silicon-based materials which may form siloxy bonds with the second metallic surface. In general, the bonding of such silicon species with the second metallic species in accordance with the present invention is stronger than similar bonds between silicon species and the surfaces of dental alloys such as gold. For the foregoing reasons, such silicon species may profitably be employed in accordance with the present invention. Among the silicon-based functionalities which may be suitable for use as bonding substituents on the adhesion promoters of the present invention are any of the wide variety of siloxy species such as trimethoxy, triethoxymethoxy, triethoxy, and many others. Accordingly, adhesion promoters having such silicone functionalities may be represented by illustrative compounds such as alpha-methacryloxypropyltrimethoxysilane.

A third alternative for the adhesion promoters in accordance with the present invention comprises those materials which are capable of coordinating with metallic atoms or ions on the surface of the second metallic species. In this regard, coordination is meant to indicate the formation of bonds of ligature as will be understood by those familiar with the field of inorganic ligand bonding. Among those compositions suitable for use as adhesion promoters in the compositions suitable for use as adhesion promoters in the present invention which employ coordination functionalities to improve the bonding ability with the second metallic surface are vanillic acid, 4-META (4-methacryloxyethoxy mellitic anhydride) and others.

Amounts of adhesion promoter are included in accordance with the present invention which are

sufficient to improve the adhesion between the opaqueing agent and the second metallic surface. When the adhesion promoter is applied to the second metallic surface prior to the application of the opaqueing agent, an excess is generally applied and unreacted material removed. When the adhesion promoter is included within the opaqueing agent itself, amounts sufficient substantially to improve the adhesion between the second metallic surface and the opaqueing layer are included therewith. In general, amounts from about 0.001% to about 20% by weight are thus included in the opaqueing agent.

An additional group of adhesion promoters which is suitable for use in connection with the practice of a particular embodiment of this invention is also known. This group comprises the polymerizable acids such as acrylic acid, methacrylic acid, and others which promoter adhesion when included as a constituent of the opaqueing agent compositions. The exact mechanism whereby the foregoing acids promote the adhesion of opaqueing agent to metallic surface is not presently understood. It is believed likely that the foregoing involves either ionic or covalent bonds, possibly through the formation of mixed organic-inorganic esters.

In order that the invention may be well understood, the following Examples are given by way of illustration only.

Example 1

A specimen metal tab 3/8×3/4" (9.4×18.8 mm) was cast in accordance with standard technique, using "Biobond"® Crown and Bridge Nickel Chrome Alloy (product of Dentsply International Inc.). The surface of the specimen was prepared by grinding with dental stones and then paascheing using an alumina laden air stream with an Air Eraser (product of Paasche Airbrush Co.) according to directions. The surface was then brush plated, using Alkaline Tin Plating Solution 5001 (product of Liquid Development Co.) according to directions, involving contacting a remote site of the specimen with a cathode and a metal stylus comprising the anode the end of which is wrapped with a cotton swab for introducing the plating solution onto the metal surface in such a way that the stylus itself does not contact the specimen. The specimen surface was then rinsed with water, then with dilute hydrogen peroxide, and again with water, and dried using Dust Chase (product of V. W. R. Scientific). (It would be preferred that after paascheing the surface be cleaned by brushing and rinsing with deionized water).

Example 2

Example 1 was modified by applying to the ground surface a dilute hydrochloric-citric acid solution having a pH of 1 while electrifying the specimen with a forward current of 4 amps per square inch at 8 volts in lieu of paascheing. An adherent layer of tin resulted when the plating procedure of Example 1 was followed.

Example 3

The surface of an article of metal can also be plated using the electroless procedure, as follows. Prepare the surface of the article by grinding and paascheing. The article would be plated by immersion in an electroless tin plating solution, such as Stanomerse® (product of Gold Rhodium Technic, Inc.) or by brushing on, causing elemental tin to deposit on the surface. Excess solution would be rinsed off with water and/or dilute hydrogen peroxide and then dried.

Example 4

A specimen prepared and plated in accordance with Example 1 was primed by applying an adhesion promoter consist of an 0.3% solution of glycerophosphoric acid dimethacrylate in ethanol, and allowing the ethanol to evaporate.

Example 5

An opaquer composition was prepared consisting of:

| % By weight | |
|---|---|
| 8.49 | Poly(methyl methacrylate-coacrylonitrile) (60:40) (molecular weight 350,000 g/mole avg. size about 50 micron) |
| 8.49 | Titanium dioxide |
| 1.87 | Rohm Plex 6661-0 |
| 4.66 | Methyl methacrylate |
| 0.03 | Dimethyl p-toluidine |
| 22.94 | Methyl ethyl ketone |
| 53.52 | Tetrahydrofuran |
| 100.00% | |

The composition was prepared by milling the polymer, titanium dioxide, methyl ethyl ketone and THF

in an Abbe mill for three hours. The remaining ingredients were added and mixed until homogeneous.

Specimens plated in accordance with Example 1 and primed in accordance with Example 4 were selected. An end portion of one surface of each specimen was coated with a layer of the opaquer of this Example, and the solvent was allowed to evaporate. An initiator solution of 1% benzoyl peroxide in diisopropyl ether was applied to the opaqued surface and allowed to dry.

A veneering composition (Biolon® Crown and Bridge Resin, product of the L. D. Caulk Co.) was applied to the opaqued surface and polymerized according to directions for use of that product at 90°C and 3 bars pressure for 25 minutes.

Example 6

Opaqued and veneered specimens prepared in accordance with Example 5 were stored in deionized water at 50°C for 94 hours. They were then thermal shock cycled thirty times by immersion first in boiling water for 30 sec, then ice water for thirty seconds. Lap shear specimens were prepared by affixing one end of a 3/8×1" (9.4×25.4 mm) piece of aluminium having a 1/8" (3.2 mm) hole drilled in the other end thereof to the opaqued and veneered surface of each specimen using Versilok®204 structural adhesive (a product of Hughson Chem. Co.). The samples were subjected to a lap shear test by clamping the uncoated surface of a specimen to one set of jaws of an Instron test unit, and attaching the free end of the aluminum piece to the other set of jaws using a loop of an orthodontic wire, and then stressing the specimens at 0.5 in/min (1.27 cm/min) to produce a lap shear failure. The specimens of this Example produced a lap shear value of above 395 lbs per square inch (27.8 kg/cm$^2$).

Example 7

An unpigmented composition was prepared consisting of:

| Percent by weight | |
|---|---|
| 4.54% | Polymethyl methacrylate-coacrylonitrile) (60:40) |
| 4.54 | Rohm Plex 6661-0 |
| 0.01 | Camphoroquinone |
| 0.05 | Methyl diethanolamine |
| 89.04 | Dichloromethane |
| 1.82 | Amyl acetate |
| 100.00% | |

The ingredients were milled for about one hour until homogeneous. Care was taken to avoid exposing the composition to bright light for any substantial length of time. The composition was applied to an end portion of several specimens of Midas Crown and Bridge Type III gold alloy prepared in accordance with Examples 1 and 4, and solvent allowed to evaporate. The coated surface was then exposed to visible light from a PrismaLite® Polymerization Unit (product of the L. D. Caulk Co.) for one minute to polymerize the composition. A self-curing acrylic veneering composition (Sevriton®, product of AD International) was applied to the coated surface and allowed to polymerize in accordance with directions for use of that product. After water storage (88 hours at 50°C) and thermal shock cycling, and preparation as in Example 6, specimens of this Example resulted in a lap shear value of 810 p.s.i. (57 kg/cm$^2$).

Example 8

An opaquer composition was prepared from:

| Percent by weight | |
|---|---|
| 16.67% | Silanated alumina |
| 49.83 | Aliphatic urethane acrylate (Uvithane®782 of Thiokol) |
| 16.50 | 1,6-Hexanediol diacrylate |
| 16.67 | Triethylene glycol dimethacrylate |
| 0.08 | Camphoroquinone |
| 0.25 | Methyl diethanolamine |
| 100.00% | |

The opaquer was applied to specimen alloy tabs of Examples 1 and 4, and after exposure to visible light from a Prisma-Lite® polymerization unit, a hard cured film was obtained. The opaqued surface is ready for the application of a commercial veneering composition, such as Biodent® K & B Plus (product of Zahnfabrik Wienand Sohne & Co., GmbH), and a satisfactory result will be obtained.

Example 9

An opaquer composition similar to that of Example 8 was prepared, except using Barium glass filler (product of Schott) in place of alumina, and applied and cured according to Example 8, resulting in a hard cured radiopaque film, ready for application of a commercial veneering composition.

Example 10

An opaquer composition was prepared consisting of:

| Percent by weight | |
|---|---|
| 10.18% | Poly(methyl methacrylate-coacrylonitrile (60:40) beads about 50 microns in diameter |
| 2.37 | Elvacite®2041 polymethyl methacrylate (DuPont) |
| 1.55 | Aerosil®R972 (DeGussa) |
| 11.78 | Titanium dioxide (No. 328-Whittaker, Clarke & Daniels) |
| 0.78 | A174 silane (Union Carbide) |
| 35.25 | Dichloromethane |
| 0.16 | Acrylic acid |
| 2.68 | Trimethylolpropane trimethacrylate |
| 100.00% | |

The dry ingredients, i.e. polymer beads, Elvacite, Aerosil and titanium dioxide, were milled in a ball mill for 45 minutes. The remaining ingredients were added and the mixture ball milled for 45 minutes.

The opaquer was applied to specimens prepared in accordance with Example 1 but the specimens were not primed as in Example 4. Biolon resin was applied in accordance with Example 5. Three such specimens were stored in 37°C water for one week, thermal shock cycled (30 cycles of 30 seconds in boiling water, 30 seconds in ice water) and prepared into lap shear specimens in accordance with Example 6.

These specimens were vacuum dyed with 1% methylene blue solution and tested for lap shear at 0.05 in/min (0.127 cm/min), obtaining an average lap shear value of 699 $lb/in^2$ (49.1 $kg/cm^2$), and resulted in a dye penetration of only 15%.

Five opaqued and veneered specimens were stored in water at 37°C for 4 weeks and lap shear tested without thermal shock cycling, and resulted in an average lap shear value of 510 $lb/in^2$ (35.9 $kg/cm^2$). These specimen exhibited no dye penetration.

Two opaqued and veneered specimens were stored in 37°C water for 12 weeks and resulted in a lap shear value of 651 $lb/in^2$ (145.77 $kg/cm^2$) without thermal shock cycling. These specimens exhibited no dye penetration and showed excellent stability.

Three opaqued and veneered specimens were stored in 37°C water for 24 weeks and resulted in a lap shear value of 1140 p.s.i. (80.15 $kg/cm^2$) without thermal shock cycling. They exhibited no dye penetration.

Example 11

A lap shear evaluation was conducted on three commercially available crown and bridge repair kits, "Denmat"®, "Fusion"® and "Metalit"®. Each was coated and veneered onto 3/8×3/4" (9.4×18.8 mm) tabs of Biobond crown and bridge alloy in accordance with manufacturers' instructions, except that for Fusion, the specimen was completed by coating with Sevriton veneering composition. The specimens were stored in 37°C water for 210 hours and thermal shock cycled. Each failed at the alloy-opaquer bond during thermal shock.

## Claims

1. A method for the construction of a dental appliance for use in an oral environment comprising:

(a) providing a shaped underlayment of a first metallic species generally inert with respect to the oral environment;

(b) plating onto at least a portion of the underlayment a layer of a second metallic species;

(c) applying to said surface portion an amount sufficient substantially to obscure the layer, of a polymerizable opaqueing agent; and

(d) polymerizing said opaqueing agent; characterized in that a surface portion of the layer of the second metallic species is oxidized, in a step (b'), prior to step (c) and that at least one of steps (b'), (c) and (d) is performed intraorally.

2. A method as claimed in claim 1 further comprising treating said plated layer with an adhesion promoter prior to said applying step.

3. A method as claimed in claim 2 wherein said adhesion promoter has the formula

$$(X)_m—R(Y)_n$$

wherein R is a hydrocarbyl group having 1 to 20 carbon atoms; X is selected from functionalities capable of forming inorganic ester bonds with said second metallic species, silicon species capable of forming siloxy bonds with said second metallic species, or ligand functionalities capable of forming coordination bonds with said second metallic species, Y is an ethylenically polymerizable group, and n and m are each integers of from 1 to 3.

4. A method as claimed in any one of the preceding claims further comprising applying to the obscured layer a pre-formed dental veneer.

5. A method as claimed in any one of claims 1—3 further comprising applying a polymerizable veneering composition to said opaqueing agent prior to said polymerizing step.

6. A method as claimed in any one of the preceding claims wherein said plating comprises electroplating.

7. A method as claimed in any one of the preceding claims wherein said opaqueing agent comprises a composition polymerizable through exposure to actinic light.

8. A method as claimed in any one of claims 1—6 wherein a polymerization catalyst is applied to the opaqueing layer subsequent to said application step.

9. A method as claimed in any one of the preceding claims wherein said opaqueing agent comprises a blend of monofunctional monomer and a di- or polyfunctional crosslinking agent for said monomer.

10. A method as claimed in any one of the preceding claims wherein said manufacture is a repair or reconstruction.

11. A method for the construction of a dental appliance for use in an oral environment comprising:

(a) providing a shaped underlayment of a first metallic species generally inert with respect to the oral environment;

(b) plating onto at at least a portion of the underlayment a layer of a second metallic species;

(c) applying to said surface portion a polymerizable opaqueing agent, said opaqueing agent including an adhesion promoter agent; and

(d) polymerizing said opaqueing agent; characterized in that a surface portion of the surface layer of the second metallic species is oxidized, in a step (b'), prior to step (c), at least one of steps (b') (c) and (d) is performed intraorally and the second metallic species has a greater tendency to bond with the polymerizable adhering agent than does the first metallic species.

**Patentansprüche**

1. Verfahren zur Herstellung einer Dentalvorrichtung für die Verwendung im Mund durch

(a) Herstellung einer geformten Unterlage aus einem ersten Metall, welches in Bezug auf die orale Umgebung inert ist;

(b) Plattierung mindestens eines Bereichs der Unterlage mit einer Schicht eines zweiten Metalls;

(c) Aufbringen eines polymerisierbaren Opakisierungsmittels in einer zur Abdeckung der Schicht im wesentlichen ausreichenden Menge auf den vorgenannten Oberflächenbereich; und

(d) Polymerisieren des Opakisierungsmittels, dadurch gekennzeichnet, daß ein Oberflächenbereich der Schicht des zweiten Metalls in Stufe (b') vor der Stufe (c) oxidiert wird und daß mindestens eine der Stufen (b'), (c) und (d) intraoral durchgeführt wird.

2. Verfahren nach Anspruch 1, dadurch gekennzeichnet, daß man vor der Aufbringstufe die Plattierungsschicht mit einem Adhäsionspromotor behandelt.

3. Verfahren nach Anspruch 2, dadurch gekennzeichnet, daß der Adhäsionspromotor folgende Formel hat

$$(X)_m—R(Y)_n,$$

wobei R einen Kohlenwasserstoffrest mit 1 bis 20 Kohlenstoffatomen bedeutet; wobei X ausgewählt ist aus funktionellen Gruppen, welche zur Bildung von anorganischen Esterbindungen mit dem zweiten Metall befähigt sind oder Siliciumgruppen, welche zur Bildung von Siloxybindungen mit dem zweiten Metall befähigt sind oder Ligantfunktionen, welche zur Bildung von Koordinationsbindungen mit dem zweiten Metall befähigt sind, wobei Y eine äthylenische polymerisierbare Gruppe ist und wobei n und m jeweils ganze Zahlen von 1 bis 3 bedeuten.

4. Verfahren nach einem der vorhergehenden Ansprüche, dadurch gekennzeichnet, daß man auf die abgedeckte Schicht eine vorgeformte Dentalauflage aufbringt.

5. Verfahren nach einem der Ansprüche 1 bis 3, wobei man eine polymerisierbare Auflagemasse dem Opakisierungsmittel vor der Polymerisationsstufe beigibt.

6. Verfahren nach einem der vorhergehenden Ansprüche, dadurch gekennzeichnet, daß die Plattierungsstufe eine Elektroplattierungsstufe umfaßt.

7. Verfahren nach einem der vorhergehenden Ansprüche, dadurch gekennzeichnet, daß das Opakisierungsmittel eine durch Belichtung polymerisierbare Masse umfaßt.

8. Verfahren nach einem der Ansprüche 1 bis 6, dadurch gekennzeichnet, daß man nach der Aufbringstufe der Opakisierungsschicht einen Polymerisationskatalysator beigibt.

9. Verfahren nach einem der vorangehenden Ansprüche, dadurch gekennzeichnet, daß das

Opakisierungsmittel eine Mischung eines monofunktionellen Monomeren und eines di- oder polyfunktionellen Vernetzungsmittels für das Monomere umfaßt.

10. Verfahren nach einem der vorangehenden Ansprüche, wobei es sich bei der Herstellung um eine Reparatur oder um einen Zahnersatz handelt.

11. Verfahren zur Herstellung einer Dentalvorrichtung für die Verwendung im Mund, umfassend die folgenden Stufen:

(a) Ausbildung einer geformten Unterlage aus einem ersten Metall, welches in Bezug auf die Umgebung des Mundes inert ist;

(b) Plattieren mindestens eines Bereichs der Unterlage mit einer Schicht eines zweiten Metalls;

(c) Aufbringen eines polymerisierbaren Opakisierungsmittels auf den Oberflächenbereich, wobei das Opakisierungsmittel einen Adhäsionspromotor umfaßt; und

(d) Polymerisieren des Opakisierungsmittels, dadurch gekennzeichnet, daß ein Oberflächenbereich der Oberflächenschicht des zweiten Metalls oxidiert wird und zwar in einer Stufe (b') vor der Stufe (c), wobei mindestens eine der Stufen (b'), (c) und (d) intraoral durchgeführt wird und wobei das zweite Metall eine größere Neigung zur Ausbildung von Bindungen mit dem polymerisierbaren Adhäsionsmittel aufweist als das erste Metall.

**Revendications**

1. Procédé de fabrication d'une prothèse dentaire, pour utilisation dans un environnement oral, consistant:

(a) à créer un substrat façonné, en une première espèce métallique, généralement inerte vis-à-vis de l'environnement oral;

(b) à déposer par métallisation, sur au moins une partie du substrat, une couche d'une deuxième espèce métallique;

(c) à appliquer sur ladite partie de surface une quantité d'un agent opacifiant polymérisable, pratiquement suffisante pour recouvrir la couche; et

(d) à polymériser ledit agent d'opacification; caractérisé en ce que, dans une étape (b') précédant l'étape (c), on oxyde une partie de la surface de la couche de la deuxième espèce métallique, et qu'au moins l'une des étapes (b'), (c) et (d) est réalisée à l'intérieur de la bouche.

2. Procédé selon la revendication 1, consistant en outre à traiter la couche métallisée avec un promoteur d'adhérence, avant ladite étape d'application.

3. Procédé selon la revendication 2, caractérisé en ce que le promoteur d'adhérence a la formule

$$(X)_m—R(Y)_n$$

dans laquelle R est un groupe hydrocarboné ayant de 1 à 20 atomes de carbone; X est choisi parmi les fonctionnalités à même de former des liaisons ester inorganique avec ladite deuxième espèce métallique, les espèces de silicium à même de former des liaisons siloxy avec ladite deuxième espèce métallique, ou des fonctionnalités du type ligand à même de former des liaisons de coordination avec ladite deuxième espèce métallique, Y est un groupe pouvant subir une polymérisation éthylénique, et n et m sont chacun des entiers de 1 à 3.

4. Procédé selon l'une quelconque des revendications précédentes, consistant en outre à appliquer sur la couche recouverte un placage dentaire préformé.

5. Procédé selon l'une quelconque des revendications 1 à 3, consistant en outre à appliquer une composition de placage polymérisable sur ledit agent opacifiant, avant ladite étape de polymérisation.

6. Procédé selon l'une quelconque des revendications précédentes, dans lequel ladite métallisation est un dépôt électrolytique.

7. Procédé selon l'une quelconque des revendications précédentes, dans lequel ledit agent opacifiant est une composition polymérisable par exposition à une lumière actinique.

8. Procédé selon l'une quelconque des revendications 1—6, dans lequel on applique un catalyseur de polymérisation à la couche opacifiante après ladite étape d'application.

9. Procédé selon l'une quelconque des revendications précédentes, dans lequel ledit agent opacifiant est constitué d'un mélange d'un monomère monofonctionnel et d'un agent de réticulation di- ou polyfonctionnel vis-à-vis dudit monomère.

10. Procédé selon l'une quelconque des revendications précédentes, dans lequel ladite fabrication est une réparation ou une reconstruction.

11. Procédé de fabrication d'une prothèse dentaire pour utilisation dans un environnement oral, consistant:

(a) à créer un substrat façonné en une première espèce métallique, généralement inerte vis-à-vis de l'environnement oral;

(b) à déposer par métallisation sur au moins une partie du substrat une couche d'une deuxième espèce métallique;

(c) à appliquer sur ladite partie de surface un agent opacifiant polymérisable, ledit agent opacifiant comprenant un agent promoteur d'adhérence; et

(d) à polymériser ledit agent opacifiant; caractérisé en ce qu'une partie de surface de la couche superficielle de la deuxième espèce métallique est oxydée, dans une étape (b') précédant l'étape (c), qu'au moins l'une des étapes (b'), (c) et (d) est réalisée à l'intérieur de la bouche, et que la deuxième espèce métallique a, par rapport à la première espèce métallique, une tendance plus grande à se lier à l'agent d'adhérence polymérisable.